# EUROPÄISCHE PATENTSCHRIFT

(11) **EP 0 579 078 B1**
(45) Veröffentlichungstag und Bekanntmachung des Hinweises auf die Patenterteilung: **06.03.1996**
(21) Anmeldenummer: 93110699.1
(22) Anmeldetag: 05.07.1993
(51) Int. Cl.: A61K 7/42

(54) **Kosmetisches Sonnenschutzmittel**
Cosmetic sunscreen
Ecran solaire cosmétique

(30) Priorität: 16.07.1992 DE 4223464
(43) Veröffentlichungstag der Anmeldung: 19.01.1994
(73) Patentinhaber: BAYER AG, D-51368 Leverkusen (DE)
(72) Erfinder: Finkel, Peter, Dipl.-Ing., D-51061 Köln (DE); Voss, Eckart, Dr., D-51375 Leverkusen (DE)

(56) Entgegenhaltungen:
- EP-A- 0 343 444
- GB-A- 2 184 356
- US-A- 5 102 654

## Beschreibung

Die vorliegende Erfindung betrifft ein neues kosmetisches Sonnenschutzmittel in Emulsionsform zur äußeren Anwendung auf der Basis von absorbierenden und reflektierenden Substanzen in Kombination mit einem neuen Radikalfängersystem, sowie ein Verfahren zu dessen Herstellung.

Zur Reduktion der für die Hautalterung und für Hautschädigungen verantwortlichen UV-Strahlen enthalten kosmetische Sonnenschutzmittel üblicherweise UV-A-und/oder UV-B-Filter. Bekannt ist aber auch der Einstz von Pigmenten, insbesondere Titandioxid-Pigmenten, als UV-reflektierende Wirkstoffe.

Vitamin-E- beziehungsweise seine Derivate werden als Radikalfänger-Wirkstoff in der Kosmetik vielfach eingesetzt. Dessen Wirkung beruht auf der Antioxidans-Wirkung in und auf der Haut (H. Möller et al. Parfümerie und Kosmetik, 68, 11, 688 (1987).

Die bekannten kosmetischen Sonnenschutzmittel sind durch eine Reihe von Nachteilen gekennzeichnet.

Die zugelassenen UV-Filter sind üblicherweise nur im UV-B-Bereich wirksam. Lediglich eine Substanzklasse, die Dibenzoylmethan-Derivate, absorbiert im gesamten UV-A-Bereich. Die beiden aus dieser Klasse zugelassenen UV-Filter weisen jedoch ein über den der anderen UV-Filter liegendes allergenes Potential auf.

Produkte, die Pigmente als Wirkstoffe enthalten, weisen ein relativ geringes Allergierisiko auf, führen jedoch aufgrund der starken Lichtreflektion und des hohen Feststoffgehaltes (make-up Effekt) zu einer niedrigen Verbraucherakzeptanz. Bei der modernen Pigmentvariante, den Titandioxyd-Mikropigmenten, ist der kosmetische Nachteil wegen der besonders geringen Teilchengröße deutlich vermindert. Einher mit der geringeren Teilchengröße geht allerdings auch ein Verlust der Wirksamkeit im UV-A-Bereich.

Die Funktion von üblichen Vitamin-E-Derivaten als Radikalfänger in kosmetischen Sonnenschutzmitteln wird durch die unzureichende und/oder inhomogene Verteilung in der Epidermis limitiert. So sind normalerweise gerade in der sensiblen Basalschicht der Epidermis nur relativ geringe Wirkstoffspiegel erzielbar, und selbst mit den heute vielfach eingesetzten Liposomen als Vehikel wird keine große Verbesserung in dieser Hinsicht erzielt.

Es wurde nun ein neues kosmetisches Sonnenschutzmittel in Emulsionsform gefunden, das neben üblichen Grund- und Hilfsstoffen eine Kombination aus wenigstens einem UV-Filter, insbesondere UV-B-Filter und mikronisiertem, stabilisiertem und im wesentlichen transparentem Zinkoxid als UV-A-Filter sowie eine Kombination aus Vitamin E- oder seinen Derivaten und 2-(Dihydroxyethyl)-2-hydroxy-6,10,14-trimethyl-pentadecan enthält. Die letztere Verbindung ist als Haar-und Hautpflegemittel bekannt (G. Erkmann et al; Seifen, Öle, Fette, Wachse 117, 10, 379 (1991). So wird diese Verbindung eingesetzt, um die Feuchtigkeit der Haare zu erhalten, das Haar vor mechanischer Beschädigung zu schützen, das Eindringen von Panthenol und Aminosäuren in den Haarschaft zu begünstigen, dem matten und geschädigten Haar Glanz zu verleihen sowie das Feuchthaltevermögen der Haut zu erhöhen und diese glatt und geschmeidig zu erhalten.

Über den Einsatz des 2-(Dihydroxyethyl)-2-hydroxy-6,10,14-trimethyl-pentadecan zusammen mit Vitamin-E-beziehungsweise seinen Derivaten und insbesondere eine damit einhergehende Depotwirkung ist bislang nichts bekannt geworden. Diese Verbindung bewirkt eine erhöhte Penetration und/oder Diffusion des Vitamin-E beziehungsweise seiner Derivate in die beziehungsweise der Haut.

Weiterhin wurde gefunden, daß man das neue, erfindungsgemäße Sonnenschutzmittel bestehend aus den wirksamen Bestandteilen wie UV-A- und UV-B-Filter, Vitamin-E- und/oder seinen Derivaten, (2-Dihydroxyethyl)-2-hydroxy-6,10,14-trimethylpentadecan sowie gegebenenfalls einen und/oder mehrere Grund- oder Hilfsstoffe erhält, wenn man die Komponenten nach den üblichen Methoden miteinander vordispergiert und verrührt und gegebenenfalls anschließend homogenisiert. Vorzugsweise wird die gesamte Herstellung in einer evakuierten Apparatur durchgeführt, um das Einarbeiten von Luft zu vermeiden.

Die Erfindung betrifft allgemein auch die neue Verwendung von 2-(Dihydroxyethyl)-2-hydroxy-6,10,14-trimethyl-pentadecan zur Erhöhung der Penetration und/oder Diffusion von Vitamin E beziehungsweise seiner Derivate in die beziehungsweise der Haut, insbesondere in kosmetischen Sonnenschutzmitteln.

Überraschenderweise zeigt das erfindungsgemäße Sonnenschutzmittel keine der oben beschriebenen Nachteile im Vergleich zu den aus dem Stand der Technik bekannten Sonnenschutzmittel-Formulierungen.

Zum Schutz vor UV-B-Strahlung enthalten die erfindungsgemäßen Rezepturen einen oder mehrere der üblicherweise verwendeten UV-B-Filter, insbesondere einem oder mehrere der zugelassenen UV-B-Filter der EG-Positivliste. Diese sind in der "vierzehnten Richtlinie 92/8/EWG der Kommission" vom 18. Februar 1992 veröffentlicht worden (siehe hierzu Amtsblatt der Europäischen Gemeinschaften ABL. Nr. L 70/23 vom 17. März 1992).

Der Schutz vor UV-A-Strahlung wird durch mikronisiertes und stabilisiertes Zinkoxid erreicht. Im Gegensatz zu Titandioxid weist dieses Zinkoxid eine deutlich größere Transparenz auf. Dies bedeutet auch eine wesentlich höhere Verbraucherakzeptanz.

Um den Einsatz von Vitamin-E und seine Derivate zu optimieren, wurden verschiedene Versuche zur Verbesserung der Penetration und Diffussion der Substanz in der Epidermis durchgeführt. Besonders überraschend war, daß eine Kombination von Vitamin E-Derivaten mit 2-(Dihydroxyethyl)-2-hydroxy-6,10,14-trimethyl-pentadecan zu einer völlig homogenen Verteilung der Vitamin E-Derivate im gesamten Bereich der Epidermis führt. Dies ist die Voraussetzung für einen optimalen Einsatz eines Radikalfängersystems in lichtexponierter Haut.

Es konnte weiterhin gezeigt werden, daß das erfindungsgemäße Sonnenschutzmittel zu einem Depot an Vitamin-Derivaten, insbesonder Vitamin E-Acetat in der Haut, besonders in den tieferen Bereichen der Epidermis, die für Alterungsprozesse wichtig sind, führt. Das ist eine ideale Voraussetzung für die Bereitstellung des Wirkstoffes. Das erfindungsgemäße kosmetische Sonnenschutzmittel stellt somit ein hochwirksames System zum Schutz vor akuten und chronischen Lichtschäden der Haut bei hervorragenden kosmetischen Eigenschaften.

Vorzugsweise werden erfindungsgemäß die üblichen Vitamin-E-Derivate eingesetzt, wobei als besonders bevorzugt die Vitamin-E-Ester aufgeführt seien. Ganz besonders bevorzugt werden Vitamin-E-Linoleat und Vitamin-E-Acetat oder deren Mischungen verwendet.

Bevorzugt ist ein neues kosmetisches Sonnenschutzmittel, das die wirksamen Bestandteile in folgender ZusammenSetzung enthält:
0,1 bis 20 % eines oder mehrerer UV-B-Filter,
0,5 bis 20 % eines mikronisierten, transparenten und stabilisierten Zinkoxids,
0,1 bis 5 % Vitamin E-Acetat,
0,1 bis 5 % 2-(Dihydroxyethyl)-2-hydroxy-6,10,14-trimethyl-pentadecan.

Weiterhin enthält das erfindungsgemäße kosmetische Sonnenschutzmittel als Grund- und/oder Hilfsstoffe einen oder mehrere der folgenden Stoffe:
Antioxidantien, Lösungsmittel, mineralische, tierische oder pflanzliche Öle oder Wachse, Fettsäuren, Fettalkohole, Fettsäureester, Fettalkoholether, oxyethylierte Fettalkohole, Lanolin oder Lanolinderivate, Siliconöle, Emulgatoren, Verdickungsmittel, Feuchthaltemittel, Farbstoffe, Puffersubstanzen, Konservierungsmittel und Parfümöle.

Das erfindungsgemäße Sonnenschutzmittel enthält besonders bevorzugt
0,5 bis 10 % eines oder mehrerer UV-B-Filter,
1 bis 10 % eines mikronisierten, transparenten und stabilisierten Zinkoxids,
0,2 bis 4 % Vitamin E-Acetat,
0,2 bis 4 % 2-(Dihydroxyethyl)-2-hydroxy-6,10,14-trimethyl-pentadecan,
sowie einen oder mehrere der oben genannten Grund- und/oder Hilfsstoffe.

Ganz besonders bevorzugt ist eine Sonnenschutzmittel-Formulierung, die
2 bis 6 % eines oder mehrerer UV-B-Filter,
3 bis 6 % eines mikronisierten, transparenten und stabilisierten Zinkoxids,
0,5 bis 2 % Vitamin E-Acetat,
0,5 bis 2 % 2-(Dihydroxyethyl)-2-hydroxy-6,10,14-trimethyl-pentadecan,
sowie einen oder mehrere der oben genannten Grund- und/oder Hilfsstoffe enthält.

Als UV-B-Filter kommen vorzugsweise alle in der EG-Positivliste genannten Verbindungen infrage. Diese umfaßt Benzylidenkampferverbindungen, p-Aminobenzoesäure sowie ihre Derivate, Cinnamate, Benzoxazol-Derivate, Benzophenonderivate und Benzotriazol-Derivate.

Vorzugsweise werden folgende UV-B-Filter für die Formulierung des erfindungsgemäßen Sonnenschutzmittels eingesetzt:
N-Propoxylierter 4-Aminobenzoesäure-ethylester (Mischung von Isomeren),
Ethoxilierter 4-Aminobenzoesäure-Ethylester,
4-Aminobenzoesäure-glycerylester,
2-Ethylhexyl-4-dimethylaminobenzoat,
2-Ethylhexylsalicyclat,
4-Methoxizimtsäure-isopentylester (Mischung von Isomeren),
2-Ethylhexyl-4-methoxycinnamat,
2-Hydroxy-4-methoxy-4'-methyl-benzophenon [Mexenon (INN)],
2-Hydroxy-4-methoxybenzophenon-5-sulfonsäure und Natriumsalz (Sulisobenzon und Natriumsalz),
α-(2-Oxoborn-3-yliden-toluen)-4-Sulfonsäure und ihre Salze,
3-(4'-Methylbenzyliden)-d,l-Campfer,
3-Benzylidencampfer,
4-Isopropyl-dibenzoylmethan,
4-Isopropylbenzylsilicyclat,
1-(4-tert,-Butylphenyl)-3-(4-methoxyphenyl)propan-1,3-dion,
2,4,6-Trianilin-(p-carbo-2'-ethylhexyl-1'-oxi)-1,3,5-triazin.

Besonders bevorzugt werden als UV-B-Filter folgende Verbindungen eingesetzt:
2-Ethoxyhexyl-p-(dimethylamino)-benzoat;
2-Ethylhexyl-p-methoxycinnamat;
3-(4'-Methylbenzyliden)-d,1-kampfer;
2-Hydroxy-5-methoxybenzophenon;
2-Hadroxy-4-methoxybenzophenon-5-sulfonsäure;
2-Phenylbenzimidazol-5-sulfonsäure.

Als UV-A-Filter eignen sich vorzugsweise mikronisierte Zinkoxid-Qualitäten mit einer Primärteilchengröße von 5 bis 100 µm, vorzugsweise 10 bis 100 µm, besonders bevorzugt 10 bis 50 µm.

Beschichtetes Material an mikronisiertem Zinkoxid und Vordispersionen desselbigen in Ölkomponenten eignen sich besonders gut zur Verarbeitung für das erfindungsgemäße kosmetische Sonnenschutzmittel.

Das erfindungsgemäße kosmetische Sonnenschutzmittel enthält neben der o. a. Wirkstoffkombination üblicherweise in kosmetischen Mitteln eingesetzte Grund- und Hilfsstoffe, insbesondere Stabilisatoren und Antioxidantien wie Butylhydroxyanisol, Butylhydroxytoluol, EDTA, Salze wie Magnesiumsulfat in Mengen von 0,02 bis 5 % u. a.

Zu den Grund- und Hilfsstoffen zählen des weiteren in der Kosmetik übliche Lösungsmittel wie Wasser bis zu 80 %, Monoalkohole, niedrige Polyalkohole mit 1 bis 6 Kohlenstoffatomen oder Mischungen davon, weiterhin Fettkörper, wie mineralische, tierische oder pflanzliche Öle wie Paraffinöl oder Wachse wie Mikrowachs, Fettsäuren, Fettalkohole, Fettsäureester wie Cetylstearyl-isononanoat und Isopropylpalmitat, Fettalkoholether, oxethylierte Fettalkohole, Lanolin und Derivate, Silikonöle in Mengen von 0,5 bis 50 %, vorzugsweise 0,5 bis 30 %, besonders bevorzugt in Mengen von 5 bis 30 %.

Das erfindungsgemäße kosmetische Sonnenschutzmittel enthält gegebenenfalls Emulgatoren in Mengen von 0,1 bis 20 %, bevorzugt in Mengen von 0,2 bis 10 %, wobei es sich um nichtionische, anionische, kationische oder amphotere Verbindungen handelt, z.B. Sterole, Polyol-Fettsäureester und -Fettalkoholether, Alkali- und Triethanolaminsalze von Fettsäuren, Natriumcetylstearylsulfat, Tetracylammoniumhalogenide, Phospholipide. Beispiele hierfür sind Glycerinsorbitanfettsäureester, Polyoxyethylenfettsäureester, Alkyltetraglykolether-o-phosphorsäureester.

Ferner können Verdickungsmittel im erfindungsgemäßen Sonnenschutzmittel eingesetzt sein. Dazu zählen Polyacrylsäurederivate, Cellulosederivate, Bentonite, Xanthanderivate, Alginate, Guarmehl und Johannisbrotmehl. Bevorzugt werden Polyacrylsäureamid oder Zinkstearat eingesetzt, in Konzentrationen von 0,02 bis 5 %, bevorzugt in Mengen von 0,1 bis 2 %.

Die erfindungsgemäße Sonnenschutzformulierung kann weitere in kosmetischen Mitteln übliche Stoffe wie Feuchthaltemittel, Farbstoffe, Puffersubstanzen, Konservierungsmittel und Parfümöle enthalten.

Dazu zählen Feuchthaltemittel in Mengen von 0,5 bis 15 %.

Als Feuchthaltemittel seien beispielhaft aufgeführt:
Niedrige Polyalkohole wie Glycerin, Propylenglykol, Butylenglykol, Sorbitol, desweiteren die 2-Pyrrolidon-5-Carbonsäure und ihr Natriumsalz, Milchsäure und ihre Salze, Harnstoff, Proteine und Proteinderivate wie Collagen, des weiteren Hyaluronsäure u. a.

Als den erfindungsgemäßen Sonnenschutzmitteln zuzusetzende Farbstoffe seien beispielhaft aufgeführt:
Farbe C.I. 16255, Farbe C.I. 61570, Farbe C.I. 42051,
Farbe C.I. 15985, Farbe C.I. 77492.

Ihre Menge beträgt ca. 0,01 bis 5,0 % der gesamten Formulierungsmenge.

Als Konservierungsmittel kommen vorzugsweise infrage:
2,4-Hexadiensäure (Sorbinsäure und ihre Salze)
4-Hydroxybenzoesäure, ihre Salze und Ester,
3-Acetyl-6-methyl-2,4(3H)-pyrandion (Dehydracetsäure) und seine Salze,
1,1-Methylen-bis-[3-(1-hydroxy-methyl-2,4-diocimidazolidin-5-yl)harnstoff],
Imidazolidinylharnstoff,
2-Phenoxy-ethanol,
Benzylalkohol.

Auch ihre Menge beträgt wie die Menge gegebenenfalls zuzusetzender Puffersubstanzen und Parfümöle 0,01 bis 5 % der gesamten Formulierungsmenge des erfindungsgemäßen Sonnenschutzmittels.

Das erfindungsgemäße kosmetische Mittel liegt als Emulsion (Creme oder Milch) vor. Die Darstellung erfolgt i.a. durch Mischen und Rühren der Komponenten, gegebenenfalls mit anschließendem Homogenisieren gegebenenfalls in einer evakuierten Apparatur.

Alle Prozentangaben im vorliegenden Test beziehen sich auf Gewichtsprozente, wenn nichts anderes angegeben ist.

Die Erfindung wird im folgenden anhand der Beispiele näher erläutert, ohne daß dies einschränkenden Charakter haben soll.

### Beispiel 1

### Wasser-in-Öl-Emulsion (Sonnencreme)

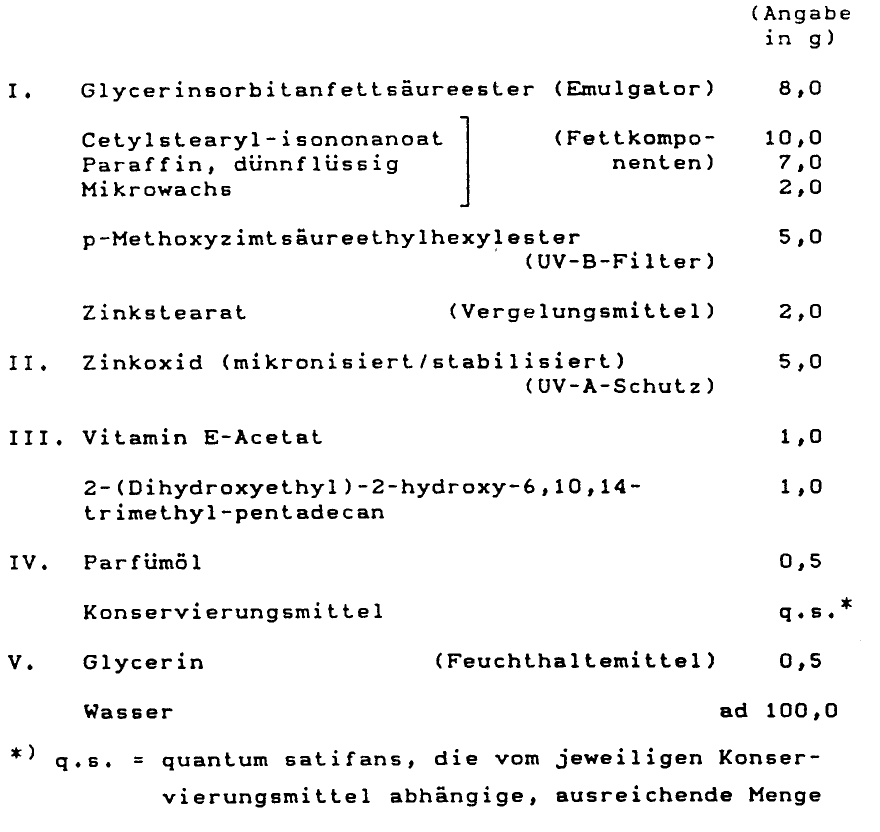

Herstellung:
Die Mischung I wird bei 75°C zum Schmelzen gebracht, II eindispergiert und die auf die gleiche Temperatur erwärmte Lösung V unter Rühren zugegeben. Unter weiterem Rühren und Homogenisieren läßt man auf 35°C abkühlen, dann gibt man die Mischung III und IV hinzu, ergänzt mit Wasser auf 100 g und läßt unter weiterem Rühren auf Raumtemperatur abkühlen Die gesamte Herstellung erfolgt in einer evakuierten Apparatur, um das Einarbeiten von Luft zu vermeiden.

### Beispiel 2

### Wasser-in-Öl-Emulsion (Sonnenmilch)

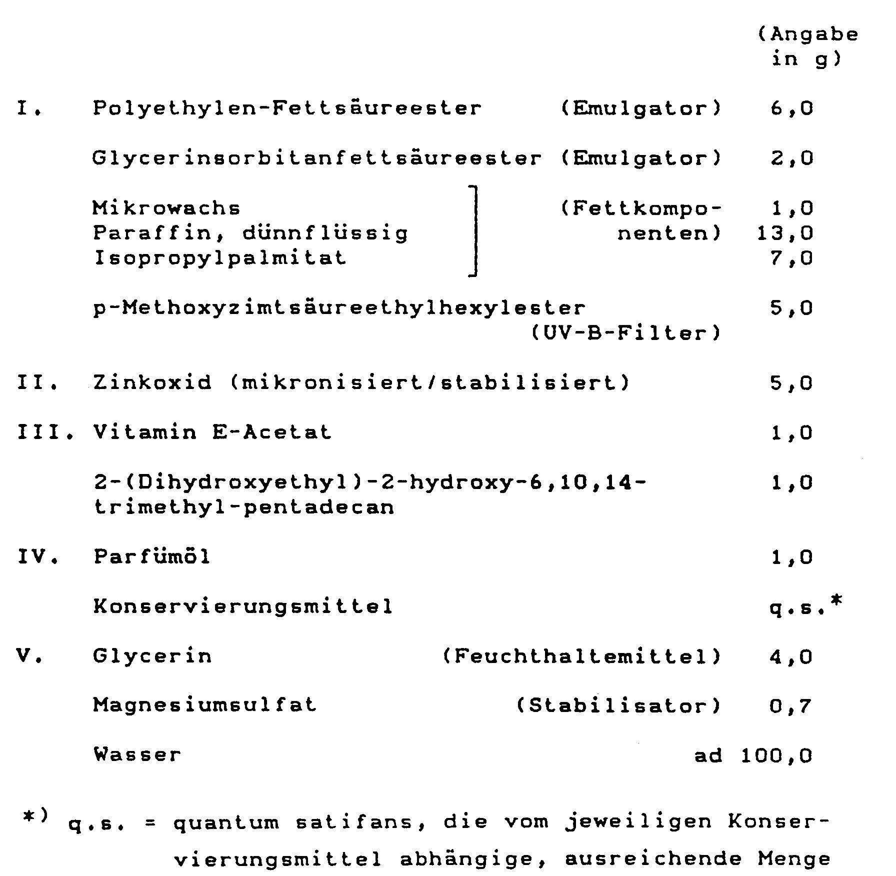

Die Herstellung der W/O-Emulsion (Hautpflegemittel) erfolgt in Analogie zu den Angaben von Beispiel 1.

### Beispiel 3

### Öl-in-Wasser-Emulsion (Sonnenmilch)

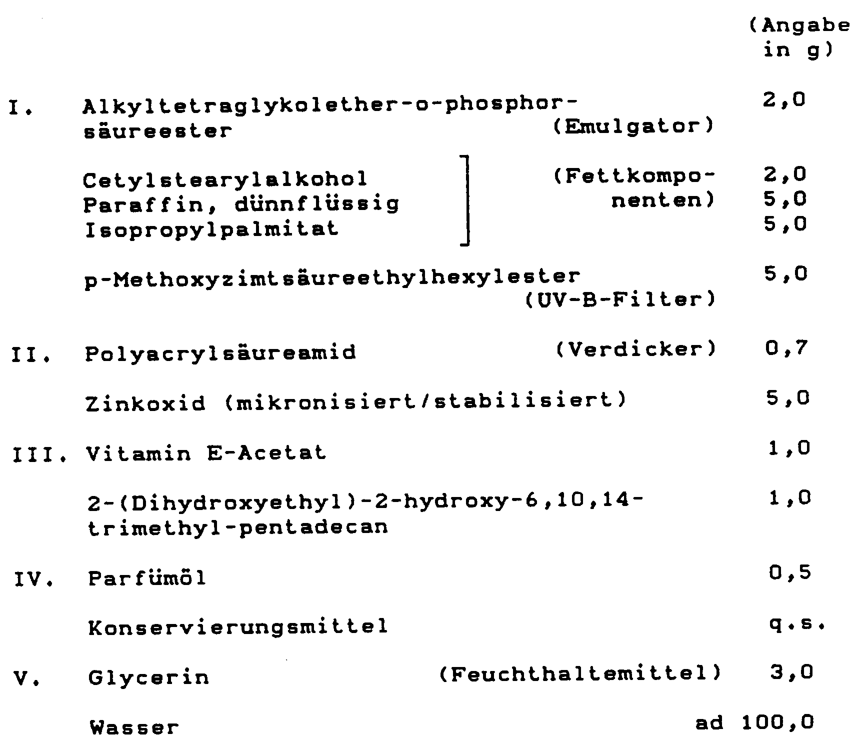

Herstellung:
Die Mischung I wird bei 75°C zum Schmelzen gebracht, II eindispergiert und der auf die gleiche Temperatur erwärmten Lösung V zugesetzt. Unter weiterem Rühren und Homogenisieren läßt man auf 35°C abkühlen, dann gibt man die Mischung III und IV hinzu, ergänzt mit Wasser auf 100 g und läßt unter weiterem Rühren auf Raumtemperatur abkühlen. Die gesamte Herstellung erfolgt in einer evakuierten Apparatur, um das Einarbeiten von Luft zu vermeiden.

### Beispiel 4

### Wasser-in-Öl-Emulsion (Sonnencreme)

| | | (Angabe in g) |
|---|---|---|
| I. | Polyethylenglycol-1-Glycerol-Sorbitan-Isostearat | 10,0 |
| | Bienenwachs | 3,0 |
| | Lanolin | 3,0 |
| | Capryl-/Caprinsäuretriglycerid | 6,0 |
| | Perhydrosqualen | 6,0 |
| | Weizenkeimöl | 3,0 |
| | p-Methoxyzimtsäureethylhexylester | 5,0 |
| II. | Zinkoxid (mikronisiert/stabilisiert) | 5,0 |
| III. | Vitamin E-Acetat | 1,0 |
| | 2-(Dihydroxyethyl)-2-hydroxy-6,10,14-trimethyl-pentadecan | 1,0 |
| | Ascorbylpalmitat | 0,1 |
| IV. | Parfümöl | q.s. |
| V. | Glycerin | 2,0 |
| | Konservierungsmittel | q.s. |
| | Magnesiumsulfat | 0,7 |
| | Wasser | ad 100,0 |

Herstellung:
Die Mischung I wird bei 75°C zum Schmelzen gebracht, II eindispergiert und die auf die gleiche Temperatur erwärmte Lösung V unter Rühren zugegeben. Unter weiterem Rühren und Homogenisieren läßt man auf 35°C abkühlen, dann gibt man die Mischung III und das Parfümöl IV hinzu, ergänzt mit Wasser auf 100 g und läßt unter weiterem Rühren auf Raumtemperatur abkühlen. Die gesamte Herstellung erfolgt in einer evakuierten Apparatur, um das Einarbeiten von Luft zu vermeiden.

### Beispiel 5

### Wasser-in-Öl-Emulsion (Sonnenmilch)

| | | (Angabe in g) |
|---|---|---|
| I. | Polyethylenglycol-1,5-polyoxyethylen-2,5-glycerol-Sorbitan-Hydroxystearat | 6,0 |
| | Cyclomethicone | 6,0 |
| | Paraffin, dünnflüssig | 6,0 |
| | p-Methoxyzimtsäureethylhexylester | 3,0 |
| II. | Zinkoxid (mikronisiert/stabilisiert) | 5,0 |
| III. | Vitamin E-Acetat | 1,0 |
| | 2-(Dihydroxyethyl)-2-hydroxy-6,10,14-trimethyl-pentadecan | 1,0 |
| IV. | Parfümöl | q.s. |
| V. | Polyethylenglycol-Sorbitol | 4,0 |
| | Magnesiumsulfat | 0,7 |
| | Konservierungsmittel | q.s. |
| | Wasser | ad 100,0 |

Die Herstellung der Wasser-in-Öl-Emulsion (Sonnenmilch) gemäß Beispiel 5 erfolgt in Analogie zu den Angaben von Beispiel 4.

## Patentansprüche

1. Kosmetisches Sonnenschutzmittel, dadurch gekennzeichnet, daß es neben üblichen Grund- und Hilfsstoffen eine Kombination aus (a) wenigstens einem UV-Filter, (b) mikronisiertem, stabilisiertem und im wesentlichen transparentem Zinkoxid, (c) Vitamin E und/oder wenigstens eines seiner Derivate und (d) 2-(Dihydroxyethyl)-2-hydroxy-6,10,14-trimethyl-pentadecan enthält.

2. Kosmetisches Sonnenschutzmittel gemäß dem Anspruch 1, welches Vitamin-E-Linoleat und/oder Vitamin-E-Acetat enthält.

3. Kosmetisches Sonnenschutzmittel gemäß Anspruch 1, dadurch gekennzeichnet, daß es gegebenenfalls neben einem oder mehreren Grund- und/oder Hilfsstoffen die wirksamen Bestandteile in folgenden Mengen enthält (%-Angabe bedeutet Gewichtsprozente):
0,1 bis 20 % eines oder mehrerer UV-B-Filter,
0,5 bis 20 % eines im wesentlichen mikronisierten, transparenten und stabilisierten Zinkoxids,
0,1 bis 5 % Vitamin E und/oder wenigstens eines Vitamin E-Derivats und
0,1 bis 5 % 2-(Dihydroxyethyl)-2-hydroxy-6,10,14-trimethyl-pentadecan.

4. Kosmetisches Sonnenschutzmittel gemäß Anspruch 1, dadurch gekennzeichnet, daß es neben einem oder mehreren Grund- und/oder Hilfsstoffen die wirksamen Bestandteile in folgender Zusammensetzung enthält (%-Angaben bedeuten Gewichtsprozente):
0,5 bis 10 % eines oder mehrerer UV-B-Filter,
1 bis 10 % eines mikronisierten, im wesent-lichen transparenten und stabilisierten Zinkoxids,
0,2 bis 4 % Vitamin E-Acetat,
0,2 bis 4 % 2-(Dihydroxyethyl)-2-hydroxy-6,10,14-trimethyl-pentadecan.

5. Kosmetisches Sonnenschutzmittel gemäß Anspruch 1, dadurch gekennzeichnet, daß es neben einem oder mehreren Grund- und/oder Hilfsstoffen die wirksamen Bestandteile in folgender Zusammensetzung enthält (%-Angaben bedeuten Gewichtsprozente):
2 bis 6 % eines oder mehrerer UV-B-Filter,
3 bis 6 % eines im wesentlichen mikronisierten, transparenten und stabilisierten Zinkoxids,
0,5 bis 2 % Vitamin E-Acetat,
0,5 bis 2 % 2-(Dihydroxyethyl)-2-hydroxy-6,10,14-trimethyl-pentadecan.

6. Kosmetisches Sonnenschutzmittel gemäß Anspruch 1, dadurch gekennzeichnet, daß es sich um Öl-in-Wasser- oder Wasser-in-Öl-Emulsionen in Milch- oder Creme-Form handelt.

7. Kosmetisches Sonnenschutzmittel gemäß der Ansprüche 1 bis 5, dadurch gekennzeichnet, daß es als Grund- und/oder Hilfsstoffe einen oder mehrere der folgenden Stoffe enthält:
Antioxidantien, Lösungsmittel, mineralische, tierische oder pflanzliche Öle oder Wachse, Fettsäuren, Fettalkohole, Fettsäureester, Fettalkoholether, oxyethylierte Fettalkohole, Lanolin oder Lanolinderivate, Siliconöle, Emulgatoren, Verdickungsmittel, Feuchthaltemittel, Farbstoffe, Puffersubstanzen, Konservierungsmittel und Parfümöle.

8. Verfahren zur Herstellung von kosmetischen Sonnenschutzmitteln gemäß der Ansprüche 1 bis 7, dadurch gekennzeichnet, daß die in Anspruch 1 unter (a) bis (d) genannten wirksamen Bestandteile mit den üblichen Grund-und Hilfsstoffen gegebenenfalls unter Vordispergieren, Rühren und/oder Homogenisieren vermischt werden.

9. Verwendung von 2-(Dihydroxy-ethyl)-2-hydroxy-6,10,14-trimethyl-pentadecan in kosmetischen Sonnenschutzmitteln zur Erhöhung der Penetration und/oder Diffusion von Vitamin-E beziehungsweise seiner Derivate in die beziehungsweise der Haut.

## Claims

1. Cosmetic sunscreen, characterised in that, apart from customary bases and auxiliaries, it contains a combination of (a) at least one UV filter (b) micronised, stabilised and essentially transparent zinc oxide, (c) vitamin E and/or at least one of its derivatives and (d) 2-(dihydroxyethyl)-2-hydroxy-6,10,14-trimethyl-pentadecane.

2. Cosmetic sunscreen according to Claim 1, which contains vitamin E linoleate and/or vitamin E acetate.

3. Cosmetic sunscreen according to Claim 1, characterised in that, apart from one or more bases and/or auxiliaries, it optionally contains the active constituents in the following amounts (% data denote percentages by weight):
0.1 to 20% of one or more UV B filters,
0.5 to 20% of an essentially micronised, transparent and stabilised zinc oxide,
0.1 to 5% of vitamin E and/or at least one vitamin E derivative
0.1 to 5% of 2-(dihydroxyethyl)-2-hydroxy-6,10,14-trimethyl-pentadecane.

4. Cosmetic sunscreen according to Claim 1, characterised in that, apart from one or more bases and/or auxiliaries, it optionally contains the active constituents in the following amounts (% data denote percentages by weight):
0.5 to 10% of one or more UV B filters,
1 to 10% of a micronised, essentially transparent and stabilised zinc oxide,
0.2 to 4% of vitamin E acetate,
0.2 to 4% of 2-(dihydroxyethyl)-2-hydroxy-6,10,14-trimethyl-pentadecane.

5. Cosmetic sunscreen according to Claim 1, characterised in that, apart from one or more bases and/or auxiliaries, it optionally contains the active constituents in the following amounts (% data denote percentages by weight):
2 to 6% of one or more UV B filters,
3 to 6% of an essentially micronised, transparent and stabilised zinc oxide,
0.5 to 2% of vitamin E acetate,
0.5 to 2% of 2-(dihydroxyethyl)-2-hydroxy-6,10,14-trimethyl-pentadecane.

6. Cosmetic sunscreens according to Claim 1, characterised in that they are oil-in-water or water-in-oil emulsions in milk or cream form.

7. Cosmetics sunscreen according to Claims 1 to 5, characterised in that it contains one or more of the following substances as bases and/or auxiliaries: Antioxidants, solvents, mineral, animal or vegetable oils or waxes, fatty acids, fatty alcohols, fatty acid esters, fatty alcohol ethers, ethoxylated fatty alcohols, lanolin or lanolin derivatives, silicone oils, emulsifiers, thickeners, humectants, colorants, buffer substances, preservatives and perfume oils.

8. Process for the production of cosmetic sunscreens according to Claims 1 to 7, characterised in that the active constituents mentioned in Claim 1 under (a) to (d) are mixed with the customary bases and auxiliaries, if appropriate with predispersion, stirring and/or homogenisation.

9. Use of 2-(dihydroxy-ethyl)-2-hydroxy-6,10,14-trimethyl-pentadecane in cosmetic sunscreens to increase the penetration and/or diffusion of vitamin E and its derivatives into or in the skin respectively.

## Revendications

1. Produit cosmétique de protection contre le soleil, caractérisé en ce qu'il contient, outre les substances de base et auxiliaires usuelles, une combinaison de (a) au moins un filtre UV, (b) un oxyde de zinc micronisé-stabilisé et essentiellement transparent, (c) de la vitamine E et/ou au moins l'un de ses dérivés, et (d) du (2-dihydroxyéthyl)-2-hydroxy-6,10,14-triméthyl-pentadécane.

2. Produit cosmétique de protection contre le soleil selon la revendication 1, qui contient du linoléate de vitamine E et/ou de l'acétate de vitamine E.

3. Produit cosmétique de protection contre le soleil selon la revendication 1, caractérisé en ce qu'il contient éventuellement, outre une ou plusieurs des substances de base et/ou auxiliaires, les constituants actifs dans les proportions suivantes (les pourcentages indiqués sont des pour-cent en poids) :
0,1 à 20% d'un ou de plusieurs filtres pour UV-B,
0,5 à 20% d'un oxyde de zinc micronisé essentiellement transparent et stabilisé,
0,1 à 5% de vitamine E et/ou d'au moins un dérivé de la vitamine E , et
0,1 à 5% de (2-dihydroxyéthyl)-2-hydroxy-6,10,14-triméthyl-pentadécane.

4. Produit cosmétique de protection contre le soleil selon la revendication 1, caractérisé en ce qu'il contient, outre une ou plusieurs des substances de base et/ou auxiliaires, des constituants actifs dans les proportions suivantes (les pourcentages indiqués sont des pour-cent en poids) :
0,5 à 10% d'un ou de plusieurs filtres pour les UV-B,
1 à 10% d'un oxyde de zinc micronisé essentiellement transparent et stabilisé,
0,2 à 4% d'acétate de vitamine E,
0,2 à 4% de (2-dihydroxyéthyl)-2 hydroxy-6,10,14-triméthyl-pentadécane,

5. Produit cosmétique de protection contre le soleil selon la revendication 1, caractérisé en ce qu'il contient, outre une ou plusieurs des substances de base et/ou auxiliaires, des constituants actifs dans les proportions suivantes (les pourcentages indiqués sont des pour-cent en poids) :
2 à 6% d'un ou de plusieurs filtres pour UV-B,
3 à 6% d'un oxyde de zinc essentiellement micronisé transparent et stabilisé,
0,5 à 2% d'acétate de vitamine E,
0,5 à 2% de (2-dihydroxyéthyl)-2-hydroxy-6,10,14-triméthyl-pentadécane.

6. Produit cosmétique de protection contre le soleil selon la revendication 1, caractérisé en ce qu'il s'agit d'une émulsion huile-dans-l'eau ou eau-dans-l'huile sous forme de lait ou de crème.

7. Produit cosmétique de protection contre le soleil selon les revendications 1 à 5, caractérisé en ce qu'il contient comme substances de base et/ou auxiliaires une ou plusieurs des substances suivantes : antioxydants, solvants, huiles ou cires minérales, animales ou végétales, acides gras, alcools gras, esters d'acides gras, éthers d'alcools gras, alcools gras oxyéthylés, lanoline ou dérivés de la lanoline, huiles de silicone, émulsifiants, agents épaississants, agents humidifiants, colorants, substances tampons, agents de conservation et huiles parfumées.

8. Procédé de préparation de produits cosmétiques de protection contre le soleil selon les revendications 1 à 7, caractérisé en ce que les constituants actifs mentionnés dans la revendication 1 sous (a) à (d) sont mélangés aux substances de base et auxiliaires usuelles, éventuellement avec prédispersion, agitation et/ou homogénéisation.

9. Utilisation du (2-dihydroxyéthyl)-2-hydroxy-6,10,14-triméthyl-pentadécane dans les produits cosmétiques de protection contre le soleil, pour augmenter la pénétration et/ou la diffusion de la vitamine E ou de ses dérivés dans la peau.
